# DEMANDE DE BREVET EUROPEEN

(11) **EP 1 512 390 A1**
(43) Date de publication de la demande: **09.03.2005**
(21) Numéro de dépôt: 04291619.7
(22) Date de dépôt: 28.06.2004
(51) Int. Cl.: A61K 7/00

(54) **Article cosmétique de traitement de la peau, des muqueuses, des cheveux ou du cuir chevelu et procédé de traitement cosmétique**

(30) Priorité: 28.07.2003 FR 0309234
(71) Demandeur: L'OREAL, 75008 Paris (FR)
(72) Inventeur: Simon, Pascal, 94320 Thiais (FR)
(74) Mandataire: Boulard, Denis

(57) **Abrégé**

La présente demande concerne un article cosmétique comprenant un support imprégné d'une composition cosmétique, ledit support comprenant : a) une structure de contact destinée à être mise au contact d'une surface mouillée sur laquelle le produit cosmétique est à appliquer, et b) une structure absorbante adjacente à la structure de contact, comprenant au moins un matériau hydroabsorbant de sorte que l'article puisse absorber au moins 10 fois son poids en eau, la structure de contact étant configurée de manière à : i) être perméable à l'eau présente sur ladite surface mouillée, et ii) former barrière au matériau hydroabsorbant.

## Description

La présente invention a trait à un article cosmétique, en particulier de traitement de la peau, des muqueuses, des cheveux ou du cuir chevelu. L'invention vise également un procédé de traitement cosmétique utilisant l'article selon l'invention.

Les lingettes cosmétiques sont généralement constituées d'un substrat en une matière d'origine naturelle ou synthétique, qui est de préférence un non tissé, mais qui peut être aussi une mousse ou un tissu, ledit substrat étant imprégné d'une composition adaptée au but recherché, par exemple le nettoyage ou le démaquillage de la peau, ou encore le soin de la peau. Ces lingettes sont utilisées couramment et sont appréciées pour leur coté pratique car elles sont jetables et sont imprégnées de la quantité nécessaire et suffisante de produit nettoyant ou traitant. L'utilisation de ces lingettes évitent la manipulation et le transport de flacons contenant les lotions ou laits.

Les lingettes imprégnées peuvent être humides ou sèches. Les lingettes sèches doivent être mouillées avant leur utilisation et peuvent par exemple être imprégnées d'une composition moussante qui génère de la mousse quand la lingette est mouillée, comme décrit par exemple dans le document US-A-4,303,543. Les lingettes humides peuvent être imprégnées d'une composition aqueuse telle qu'une lotion démaquillante ou qu'un lait démaquillant par exemple, et, elles sont directement appliquées sur le visage ou le corps. Elles peuvent également être imprégnées d'une composition anhydre comportant par exemple un mélange d'huiles et de tensioactifs, et, la lingette est alors soit directement utilisée sur le visage ou le corps, soit préalablement humidifiée par un peu d'eau afin d'émulsionner le mélange huiles / tensioactifs avant l'application sur la peau, comme décrit par exemple dans le document US-A-6,136,775.

Dans la demande FR 02/11607 déposée le 19 septembre 2002, la demanderesse a illustré un article cosmétique qui peut être notamment sous forme d'une lingette ou d'un gant, et que l'on applique sur peau mouillée, après le bain ou la douche.

Le passage du gant sur la peau mouillée émulsionne l'huile contenue dans le gant et la dépose sous la forme d'un lait corps hydratant onctueux et doux.

Selon un exemple de cette demande, le gant est constitué d'un non-tissé très absorbant, et il présente alors en outre l'avantage de permettre en même temps le séchage de la peau.

Le problème posé par la mise en oeuvre de l'exemple décrit dans cette demande tient au fait que, en dépit de leur bonne capacité à absorber de l'eau, de tels articles, une fois mouillés, ont tendance en réponse à leur frottement sur la peau, à libérer sur la peau des particules du matériau super absorbant qu'ils contiennent. Ces particules, gorgées d'eau ont un diamètre de 1 mm ou plus. Il résulte de leur contact avec la peau, une impression désagréable, jugée rédhibitoire par bon nombre d'utilisateurs.

En outre, la qualité de l'essuyage est jugée non satisfaisante, en raison du contact mouillé de l'article sur la peau lorsque celui-ci est gorgé d'eau.

Aussi, est-ce un des objets de l'invention que de réaliser un procédé et un article cosmétique résolvant en tout ou partie les problèmes discutés ci-avant.

C'est en particulier un objet de la présente invention que de réaliser un article destiné à la mise en oeuvre d'un traitement cosmétique sur peaux ou cheveux mouillés, et qui en outre permet un essuyage satisfaisant de la surface sur laquelle le traitement cosmétique est appliqué.

C'est un autre objet que de réaliser un tel article en améliorant les qualités sensorielles du contact entre l'article et la surface sur laquelle le traitement est appliqué, en particulier la peau.

D'autres objets encore apparaîtront dans la description détaillée qui suit.

Selon l'invention, ces objets sont atteints en réalisant un article cosmétique comprenant un support imprégné d'une composition cosmétique, ledit support comprenant :
a) une structure de contact dont une face est destinée à être mise au contact d'une surface mouillée sur laquelle le produit cosmétique est à appliquer, ladite face étant constituée au moins en partie d'un matériau hydrophobe, et
b) une structure absorbante adjacente à la structure de contact, comprenant au moins un matériau hydroabsorbant de sorte que l'article puisse absorber au moins 10 fois son poids en eau,
la structure de contact étant configurée de manière à :
i) être perméable à l'eau présente sur ladite surface mouillée, et
ii) former barrière au matériau hydroabsorbant, pour l'empêcher, en particulier lorsqu'il est gorgé d'eau, de venir au contact de ladite surface sur laquelle le produit est à appliquer.

On réalise ainsi un article avec un fort pouvoir absorbant, ce qui lui confère des bonnes propriétés d'essuyage, tout en solutionnant le problème discuté ci-avant et relatif à l'impression désagréable résultant du contact entre la peau et les particules ou fibres de matériau superabsorbant qui peuvent se détacher de la structure absorbante sous l'effet des contraintes résultant de la friction entre l'article et la surface à traiter.

En effet, la structure de contact fait barrière et maintient à distance de la peau toute particule ou fibre gorgée d'eau qui pourrait se détacher de la structure absorbante.

L'effet barrière aux particules ou fibres superabsorbantes est obtenu, en fonction des caractéristiques dimensionnelles de ces dernières, en jouant notamment sur la densité de la structure de contact et sur son épaisseur. Ces derniers sont choisis de sorte que les espaces que définit la structure perméable soient suffisamment petits comparativement à la taille des particules hydroabsorbantes pouvant être libérées lors de l'utilisation de l'article, de manière à les empêcher de traverser la structure de contact et de venir au contact de la peau.

La caractéristique relative au caractère hydrophobe du matériau formant tout ou partie de la face destinée à être mise au contact de la surface à traiter, entraine une faible hydrophilie de la structure de contact perméable, ce qui favorise la sensation d'un contact sec entre la peau et l'article cosmétique. En effet, la structure de contact est perméable à l'eau dans la mesure où elle laisse passer l'eau mais ne la retient pas ou peu c'est à dire qu'elle ne l'absorbe pas.

A titre d'exemple, la structure de contact est constituée d'un non tissé dont le grammage est compris entre 10 g/m² et 100 g/m², et de préférence, entre 15 g/m² et 60 g/m². L'épaisseur de la structure de contact est quant à elle comprise entre 0,3 mm et 1,5 mm, et de préférence, entre 0,4 mm et 0,6 mm.

La capacité d'absorption de l'article est mesurée selon la norme EDANA 10.4-02.

La surface mouillée sur laquelle est appliquée le traitement cosmétique peut être notamment la peau, les muqueuses, les cheveux ou le cuir chevelu.

Avantageusement, la face de la structure de contact destinée à être mise au contact de la peau est formée au moins en partie de fibres d'au moins un matériau hydrophobe, notamment de polyéthylène, de polypropylène ou de polyéthylène téréphtalate, pouvant avoir fait l'objet d'un traitement de type ensimage.

Elle est de préférence lisse et douce au contact sur la peau.

A titre d'exemple, la structure de contact est constituée d'un non tissé cardé ou extrudé ("spunlaid") puis thermolié en polypropylène ou en polyéthylène terephtalate ensimé. Le thermoliage peut être obtenu par exemple par calandrage ou par air chaud ("air-through bonded").

De telles structures de contact sont commercialisées notamment sous les références PPTH 23 EIS à 23 g/m², PPTH 20 EIS à 20 g/m² et PPSS 15 EIS à 15 g/m² par la Société DOUNOR, sous la référence COMFORT WHITE HYDROPHILIC© à 10 g/m² par la Société FIBERTEX, sous la référence 111 018 HI à 18 g/m² par la Société FIBERWEB, ou sous les références SAWAVLIES© 34/00/61 et 34/00/53 respectivement à 20 g/m² et 22 g/m² par la Société SANDLER.

Selon un autre mode de réalisation, la structure de contact comprend une première face destinée à être mise au contact de la surface à traiter, et une seconde face opposée à la première, la perméabilité à l'eau de la structure de contact, dans le sens de la première face vers la seconde, étant supérieure à la perméabilité de ladite structure dans le sens inverse, la structure absorbante étant disposée du côté de la seconde face.

Cette caractéristique limite le retour d'eau depuis la structure absorbante vers la structure de contact, ce qui améliore encore la sensation de contact sec produite par l'article cosmétique selon l'invention.

La première face peut être formée d'un non-tissé hydrolié, notamment à base de polyéthylène ou de polypropylène, la seconde face étant formée d'un film microperforé, notamment en polyéthylène. Le film microperforé de polyéthylène peut être extrudé ou thermocollée sur le non tissé. De tels matériaux peuvent être obtenus notamment auprès de la Société ACE SA (filiale du Groupe Rheinsiche Kunstoff Werke AG.).

Le matériau utilisé pour réaliser la structure de contact est en outre choisi de manière à assurer le contact doux requis généralement par l'application d'un produit cosmétique.

Avantageusement encore, l'article cosmétique selon l'invention comprend au moins une couche destinée, notamment par capillarité, à favoriser la répartition de l'eau traversant la structure de contact sur la structure absorbante, ladite couche étant soit formée par la structure de contact, soit formée par une structure additionnelle située entre la structure de contact et la structure absorbante.

Cette caractéristique est tout particulièrement avantageuse pour cette application où se combine traitement cosmétique et essuyage de la surface à traiter, dans la mesure où cette dernière fonction implique généralement un mouvement de translation sur la peau mouillée. Il en résulte alors une mise en contact avec l'eau plus abondante pour la partie avant de l'article (relativement au mouvement sur la peau mouillée), que celle de la partie arrière de l'article. De ce fait, l'imprégnation de la structure absorbante est très inégale. L'efficacité de l'article n'est pas optimale.

La couche de répartition/distribution est de préférence une couche de faible densité, d'un matériau hydrophobe tel que du Polypropylène ou de Polyéthylène téréphtalate et est généralement un non tissé obtenu par cardage et consolidation thermique à chaud (Air-through bonded). Cette structure permet aux fibres, relativement aérées, de véhiculer l'eau par capillarité sur l'intégralité de la surface de l'article et assurer ainsi une bonne distribution de l'eau sur toute la surface de l'article. L'imprégnation en eau de la structure absorbante s'en trouve plus uniforme.

A titre de matériaux pouvant être utilisés pour faire office de couche de répartition/distribution, on peut citer les non tissés vendus sous les références commerciales "dry web PROEF 12-012" (25 g/m² à base de fibres bi-composant en Polypropylène et polyéthylène téréphtalate ), "dry web TR2" (40 g/m² à base de fibres de Polyéthylène téréphtalate trilobées), ou "dry web T15 PROEF 15-029" (45 g/m² à base de fibres de Polyéthylène téréphtalate de fin denier), toutes commercialisées par la Société Libeltex. On peut citer également les références SAWABOND 4131 à 35 g/m² et 05/01/51 à 45 g/m² vendues par la Société SANDLER.

Afin de combiner dans une seule structure, à savoir le voile de contact, les propriétés de douceur sur la peau et de répartition rapide et efficace de l'eau lors de l'utilisation de l'article, on préfèrera des fibres bi-composantes pour favoriser la douceur ainsi que la soudabilité (notamment lorsque l'on veut réaliser une structure sous forme d'un gant par exemple), des fibres de gros denier pour favoriser un passage rapide de l'eau vers la structure absorbante. On pourra également ajouter une faible proportion de fibres de viscose pour favoriser un bon drainage.

A titre d'exemples, le matériau hydroabsorbant contenu dans la structure absorbante est sous forme d'une poudre, notamment de glycolate sodique d'amidon réticulé, ou d'amidon greffé par du polyacrylate de sodium, ou d'un copolymère d'acide acrylique/acrylate de sodium réticulé, comme par exemple le PRIMOGEL© commercialisé par la société AVEBE, le SANWET© commercialisé par la société BASF.

La poudre est disposée à l'intérieur d'une structure formée d'au moins une couche perméable à l'eau. De manière plus spécifique, ladite structure peut être un non tissé à une seule couche de fibres aiguilletées en présence de poudre super absorbante.

De manière plus spécifique, la poudre super absorbante peut être incorporée par un procédé électrostatique au sein d'un non tissé déjà formé par exemple par cardage puis consolidation par hydroliage ou aiguilletage.

Alternativement, le non tissé comprend deux feuilles superposées délimitant entre elles au moins une poche contenant ladite poudre. Il peut s'agir d'une poche unique entre les deux feuilles, ou d'une pluralité de poches réparties sur la totalité de la surface de le structure absorbante.

A titre de non tissés pouvant être utilisés pour la réalisation de la structure absorbante utilisable selon l'invention, on peut citer notamment ceux commercialisés sous les dénominations Dritex 120NN42 et 130WNNF60 par la société Georgia Pacific, et ceux commercialisés sous les dénominations HY0201040, HY0101046, HY0101100 ou HY0301038 par la société BBA.

La poudre peut être mélangée avec des fibres, notamment de cellulose provenant notamment par exemple de pâte à papier. De telles structures absorbantes avec une poudre en mélange avec des fibres, sont parfois désignées sous le terme "fluff" (matériau comprenant un mélange de poudre super absorbante et de fibres de cellulose sans traitement final de liage). De par sa structure le "fluff" manque de tenue et il est généralement maintenu entre deux couches de non tissés. De tels produits sont commercialisés notamment par les Sociétés Georgia Pacific (USA), Tembec et Tartas (France).

Selon une autre alternative, le matériau hydroabsorbant est sous forme de fibres. De telles fibres peuvent être cardées et consolidées par aiguilletage. On citera notamment :
i) des fibres d'un copolymère d'acrylate comme par exemple les non-tissés commercialisés par la société OASIS sous les références 2130 (200 g/m2, 50% fibres super absorbantes/50% fibres de PET cardées et aiguilletées), 2160 (145 g/m2, 30% fibres super absorbantes/70% fibres de PET cardées aiguilletées) ou 2091 (30% fibres super absorbantes/70% pulp et fibres bi-composantes aérodéposées ("Airlaid") et thermoliées), ou
ii) des fibres de carboxyméthyl cellulose, ou d'alginate toutes les deux commercialisées par la société ACORDIS.

Avantageusement, la structure absorbante est configurée de manière à ce que l'article puisse absorber au moins 15 fois son poids en eau, et mieux encore, au moins 20 fois son poids en eau. Typiquement, l'article doit pouvoir absorber 50 g ou plus.

La composition cosmétique utilisée dans l'article selon l'invention est destinée au traitement de la peau, des muqueuses, des cheveux, ou du cuir chevelu.

De préférence, l'article est essentiellement anhydre. Par "essentiellement anhydre" on entend " qui ne contient pas d'eau ou contient moins de 10 % d'eau, et de préférence moins de 5 % d'eau". La quantité d'eau dans l'article peut donc aller de 0 à 10 % et de préférence de 0 à 5 % du poids total de l'article.

La composition cosmétique peut contenir au moins un agent choisi parmi les auto-bronzants, les amincissants, les filtres solaires, les hydratants, les agents chauffants, les agents pour le démêlage des cheveux, les agents conditionnant de la peau ou du cheveu, les agents kératolytiques, les agents éclaircissants de la peau comme les dépigmentants, ou les agents de coloration du cheveu. L'article peut être utilisé sur l'ensemble du corps ou de la chevelure mais peut également servir pour un traitement local.

La composition peut être liquide, c'est-à-dire qu'elle présente généralement une viscosité inférieure à 150 mPa.s et plus préférentiellement inférieure à 100 mPa.s. Cette viscosité va de préférence de 1 mPa.s à 100 mPa.s, mesurée à la température ambiante (environ 25°C) avec un appareil RHEOMAT RM 180, mobile 1 ou 2 selon la viscosité du liquide. Alternativement, la composition est sous forme d'une poudre

L'article peut avoir toute taille et toute forme appropriées au but recherché. Il peut ainsi avoir par exemple la forme d'une lingette ou d'une petite serviette, notamment de forme sensiblement rectangulaire, ou la forme d'un gant ou d'une moufle, faciles à enfiler sur la main, ou une forme d'une compresse ronde. Il a généralement une surface comprise entre 0,005 m² et 0,1 m², de préférence entre 0,01 m² et 0,07 m².

Le taux d'imprégnation de la composition sur le support va généralement de 10 à 1500 %, de préférence de 50 à 500 % et encore mieux entre 70 et 250 %. Les techniques d'imprégnation des substrats par des compositions sont bien connues dans ce domaine et sont toutes applicables à la présente invention. En général, la composition d'imprégnation est ajoutée au substrat par une ou plusieurs techniques comprenant l'immersion, l'enduction, la vaporisation, le dépôt par voie électrostatique, ou le saupoudrage (notamment avant soudage de la structure absorbante à la structure de contact).

Du côté opposé à la structure de contact, la structure absorbante peut comporter au moins une couche destinée à être au contact d'une main manipulant l'article, ladite couche étant notamment en polypropylène, en viscose, ou en polyéthylène téréphtalate. A titre d'exemple plus spécifique, la couche de contact avec la main peut être constituée d'un non tissé de fibres de polypropylène hydrophobe cardées ou sous forme de filaments déposés en couche uniforme par la technologie dite "Spunlaid", puis thermoliés. Pour cette couche, on recherche en général un contact sec et un toucher agréable.

Cette structure destinée à être au contact de la main peut en outre stopper la diffusion du liquide à travers la couche absorbante dans le cas où celle-ci ne parvient à contenir toute l'eau recueillie. Elle peut aussi servir à éviter toute perte de poudre ou de fibres de la couche absorbante lors de l'utilisation.

Comme produits intéressants on peut citer les références commerciales PPSMS/17/E/O/S et PPSS/17/E/O/S à 17 g/m² vendues par la Société DOUNOR, la référence commerciale COMFORT WHITE HYDROPHOBIC© à 15 g/m² vendue par la Société FIBERTEX, la référence 197DIMSET020HOB à 20 g/m² vendue par la Société FIBERWEB et, la référence SAWABOND© 4111 à 22 g/m² vendue par la Société SANDLER.

Afin de rendre l'article réversible, c'est à dire utilisable des deux cotés, on peut entrevoir d'utiliser la structure de contact de chaque coté de la structure absorbante. Cette option sera particulièrement intéressante à poursuivre dans le cas où l'article est configuré sous forme d'une serviette ou d'une lingette.

Selon un autre aspect de l'invention, on réalise un procédé de traitement cosmétique de la peau, des muqueuses, des cheveux ou du cuir chevelu consistant à :
a) mouiller la surface à traiter ; et
b) essuyer ladite surface au moyen d'un article selon l'invention.

Par "traitement cosmétique" on entend un traitement effectué avec au moins un produit cosmétique. Par « produit cosmétique », on entend un produit tel que défini dans la Directive 93/35/CEE du Conseil du 13 juin 1993.

### Exemple de gant de soin pour le corps :

La composition utilisée pour la réalisation de cet exemple est décrite ci-dessous :

| ***Huiles*** | |
|---|---|
| | 25 |
| Ethylhexyl palmitate | |
| Huile de Parleam | 20 |
| Huile de rosier muscat | 5 |

| ***Tensioactifs*** | |
|---|---|
| | 10 |
| PEG-20 glyceryl tri-isostéarate | |

| ***Actifs*** | |
|---|---|
| | 40 |
| Glycérine | |

La composition selon cet exemple est préparée selon le procédé suivant :

On commence par préparer le mélange d'huiles, puis on y incorpore les tensioactifs à température ambiante ou à chaud selon qu'ils se présentent sous forme liquide ou solide, et on incorpore ensuite dans le mélange obtenu, l'agent gélifiant hydrophile et les adjuvants.

Le taux d'imprégnation de la composition sur l'article est de 200%. L'article est configuré sous forme d'un gant.

Le gant est constitué de deux structures multi-couches identiques formées à partir de non tissés, les structures étant découpées à la forme désirée et soudées, collés ou cousues à la périphérie.

A l'utilisation, après la douche ou le bain, le gant est passé sur la peau mouillée, sur l'ensemble du corps. Le passage du gant sur la peau mouillée émulsionne l'huile contenue dans le gant et la dépose sous la forme d'un lait corps hydratant onctueux et doux.

### Exemple de serviette conditionneuse des cheveux

La composition contient un mélange d'alcool cetylstearylique et de methosulfate de N,N-distearoyl oxyehtyl N-methyl N-hydroxyethyl ammonium, 25/75.

Le produit est incorporé dans l'article soit sous forme pure en poudre, soit sous forme prédiluée dans de l'eau et l'article est ensuite séché totalement ou partiellement de sorte à avoir un article sensiblement sec au toucher.

### Exemple de serviette hydratante corps.

La composition contient :
Glycérol : qsp 100
Aloe Vera : 0,01%
Menthol : 0,2%
Extrait d'hamamélis (à 1 % dans l'eau) : 1 %

Les ingrédients sont mélangés à température ambiante. Le taux d'imprégnation de la composition sur l'article est de 200%. L'article est configuré sous forme d'une petite serviette ou d'un gant.

### Exemple de gant autobronzant

La composition utilisée selon cet exemple contient :
Acide B,B' camphosulfonique [1, 4 divinylbenzène] en solution aqueuse à 33% : 6%
Hydroxyde de sodium : 0.28%
Glycérol : 70,72%
Eau : 5%
Citrate de trialkyle : 8
Dihydroxyacetone : 10%

Son mode de préparation identique à celui de l'exemple précédent.

Le tableau ci-après décrit divers empilements de couches pouvant former, soit l'article directement dans le cas d'une lingette ou d'une serviette, soit l'une au moins des faces de l'article lorsque celui-ci est configuré sous forme d'un gant.

| 1 | 2 | 3 | 4 | 5 | 6 | 7 | 8 | 9 | 10 | 11 | 12 | 13 | 14 |
|---|---|---|---|---|---|---|---|---|---|---|---|---|---|
| A1 | A1 | A1 | A1 | A1 | A1 | A1 | A1 | A1 | A1 | A2 | A2 | A2 | A2 |
| | | | B2 | B2 | B2 | | | B2 | B2 | | B2 | B3 | B4 |
| C2 | C3 | C4 | C2 | C3 | C4 | C3 | C4 | C3 | C4 | C3 | C3 | C3 | C3 |
| D2 | D2 | D2 | D2 | D2 | D2 | D1 | D1 | D1 | D1 | D2 | D2 | D2 | D2 |

A1-A2 désignent la structure de contact de l'article.

B2-B4 désignent la couche de répartition/distribution présente dans certains des modes de réalisation listés ci-dessus.

C2-C4 désignent la structure absorbante.

D1-D2 désignent la couche destinée au contact avec la main.

Des références commerciales/techniques correspondant à chacune des lettres A1-A2, B2-B4, C2-C4, et D1-D2 sont exemplifiées ci-après.

A1 : PPTH20EIS à 20 g/m² de chez DOUNOR
A2 : PPSS15EIS à 15 g/m² de chez DOUNOR
B2 : Dry web T15 PROEF 15-029" à 45 g/m² de chez LIBELTEX
B3 : Dry web PROEF 12-012" à 25 g/m² de chez LIBELTEX
B4 : Dry web TR2" à 40 g/m² de chez LIBELTEX
C2 : Référence 2160 à 145 g/m2 de chez OASIS
C3 : Carboxymethyl cellulose aiguilletée à 90 g/m²
C4 : Alginate aiguilleté à 150 g/m²
D1 : PPSMS/17/E/O/S à 17 g/m² de chez DOUNOR
D2 : PPSS/17/E/O/S à 17 g/m² de chez DOUNOR

## Revendications

1. Article cosmétique comprenant un support imprégné d'une composition cosmétique, ledit support comprenant :
a) une structure de contact dont une face, formée au moins en partie d'un matériau hydrophobe, est destinée à être mise au contact d'une surface mouillée sur laquelle le produit cosmétique est à appliquer, et
b) une structure absorbante adjacente à la structure de contact, comprenant au moins un matériau hydroabsorbant de sorte que l'article puisse absorber au moins 10 fois son poids en eau,
la structure de contact étant configurée de manière à :
i) être perméable à l'eau présente sur ladite surface mouillée, et
ii) former barrière au matériau hydroabsorbant, pour l'empêcher, en particulier lorsqu'il est gorgé d'eau, de venir au contact de ladite surface sur laquelle le produit est à appliquer.

2. Article cosmétique selon la revendication 1 **caractérisé en ce que** la structure de contact est formée au moins en partie de fibres d'au moins un matériau hydrophobe, notamment de polyéthylène, de polypropylène ou de polyéthylène téréphtalate.

3. Article cosmétique selon la revendication 1 ou 2 **caractérisé en ce que** la structure de contact :
i) présente un grammage compris entre 10 g/m² et 100 g/m², de préférence, entre 15 g/m² et 60 g/m² ; et
ii) a une épaisseur comprise entre 0,3 mm et 1,5 mm, et de préférence, entre 0,4 mm et 0,6 mm.

4. Article cosmétique selon l'une quelconque des revendications 1 à 3 **caractérisé en ce que** ladite structure de contact comprend une première face et une seconde face opposée à la première, la perméabilité à l'eau de la structure de contact, dans le sens de la première face vers la seconde, étant supérieure à la perméabilité de ladite structure dans le sens inverse, la structure absorbante étant disposée du côté de la seconde face.

5. Article cosmétique selon la revendication 4 **caractérisé en ce que** la première face est formée d'un non tissé, notamment à base de polyéthylène ou de polypropylène, la seconde face étant formée d'un film microperforé, notamment en polyéthylène.

6. Article cosmétique selon l'une quelconque des revendications 1 à 5 **caractérisé en ce qu'**il comprend au moins une couche destinée, notamment par capillarité, à favoriser la répartition de l'eau traversant la structure de contact sur la structure absorbante, ladite couche étant soit formée par la structure de contact, soit formée par une structure additionnelle située entre la structure de contact et la structure absorbante.

7. Article cosmétique selon la revendication 6 **caractérisé en ce que** la couche est formée d'un matériau fibreux hydrophobe, notamment à base de polypropylène ou de polyéthylène téréphtalate.

8. Article cosmétique selon l'une quelconque des revendications 1 à 7 **caractérisé en ce que** le matériau hydroabsorbant est sous forme d'une poudre, notamment de glycolate sodique d'amidon réticulé, ou d'amidon greffé par du polyacrylate de sodium, ou d'un copolymère d'acide acrylique/acrylate de sodium réticulé.

9. Article cosmétique selon la revendication 8 **caractérisé en ce que** la poudre est disposée à l'intérieur d'une structure formée d'au moins une couche perméable à l'eau.

10. Article cosmétique selon la revendication 9 **caractérisé en ce que** ladite structure est un non tissé à une seule couche, ou à deux couches superposées délimitant entre elles au moins une poche contenant ladite poudre.

11. Article cosmétique selon la revendication 9 ou 10 **caractérisé en ce que** la poudre est en mélange avec des fibres, notamment de cellulose.

12. Article cosmétique selon l'une quelconque des revendications 1 à 7 **caractérisé en ce que** le matériau hydroabsorbant est sous forme de fibres, notamment d'un copolymère d'acrylate, de carboxyméthyl cellulose, ou d'alginate.

13. Article cosmétique selon l'une quelconque des revendications précédentes **caractérisé en ce que** la structure absorbante est configurée de manière à ce que l'article puisse absorber au moins 15 fois son poids en eau, et mieux encore, au moins 20 fois son poids en eau.

14. Article cosmétique selon l'une quelconque des revendications qui précèdent **caractérisé en ce que** la composition cosmétique est destinée au traitement de la peau, des muqueuses, des cheveux, ou du cuir chevelu.

15. Article cosmétique selon l'une quelconque des revendications précédentes **caractérisé en ce qu'**il est essentiellement anhydre.

16. Article cosmétique selon la revendication 14 ou 15 **caractérisé en ce que** la composition cosmétique contient au moins un agent choisi parmi les filtres solaires, les auto-bronzants, amincissants, hydratants, agents chauffant, les agents pour le démêlage des cheveux, les agents conditionnant de la peau ou du cheveu, les agents kératolytiques, les agents éclaircissants de la peau comme les dépigmentants, ou les agents de coloration du cheveu.

17. Article cosmétique selon l'une quelconque des revendications précédentes **caractérisé en ce que** du côté opposé à la structure de contact, la structure absorbante est adjacente à au moins une couche destinée à être au contact d'une main manipulant l'article, ladite couche étant notamment en polypropylène, en viscose, ou en polyéthylène téréphtalate.

18. Article cosmétique selon l'une quelconque des revendications précédentes **caractérisé en ce que** l'imprégnation de l'article par la composition cosmétique se fait par pulvérisation, immersion, saupoudrage, enduction, ou par voie électrostatique.

19. Article cosmétique selon l'une quelconque des revendications qui précèdent **caractérisé en ce qu'**il est configuré sous forme d'un gant, d'une moufle, d'une serviette, ou d'une lingette, notamment de forme sensiblement rectangulaire.

20. Procédé de traitement cosmétique de la peau, des muqueuses, des cheveux ou du cuir chevelu consistant à :
a) mouiller la surface à traiter, et
b) essuyer ladite surface au moyen d'un article selon l'une quelconque des revendications 1 à 19.
